# EUROPEAN PATENT SPECIFICATION

(11) **EP 4 609 909 B1**
(45) Date of publication and mention of the grant of the patent: **04.11.2026**
(21) Application number: 25190122.9
(22) Date of filing: 18.09.2020
(51) Int. Cl.: A61N 1/36, A61N 1/372, A61N 1/375, A61N 1/05, A61N 1/378

(54) **IMPLANTABLE DEVICE FOR ELECTRICAL NERVE STIMULATION TO CORRECT SLEEP BREATHING**
IMPLANTIERBARE VORRICHTUNG ZUR ELEKTRISCHEN NERVENSTIMULATION ZUR KORREKTUR DER SCHLAFATMUNG
DISPOSITIF IMPLANTABLE POUR LA STIMULATION ÉLECTRIQUE DES NERFS AFIN DE CORRIGER LA RESPIRATION PENDANT LE SOMMEIL

(43) Date of publication of application: 03.09.2025
(62) Document number(s) of the earlier application(s) in accordance with Art. 76 EPC: 20775617.2 / 4 213 930
(73) Proprietor: Nyxoah SA, 1435 Mont-St-Guibert (BE)
(72) Inventor: VOSSENBERG, Marcel, 2860 Katelijne-Waver (BE); BERVOETS, Wim, 2610 Wilrijk (BE); RAJE, Milind Chandrakant, Wentworthville, 2145 (AU); VAN DEN HEUVEL, Koen Erik, 2540 Hove (BE); VERMEIREN, Jan, 2530 Boechout (BE)
(74) Representative: Brantsandpatents bv

(56) References cited:
- US-A1- 2014 031 840
- US-A1- 2019 126 051

## Description

### Technical Field

The disclosed subject matter described hereafter refers to a device for electrical nerve stimulation. Furthermore, reference is made to a use of the device for electrical nerve stimulation to correct sleep disordered breathing.

Neural modulation, e.g. electrical stimulation of nerves, is known in the prior art as a reliable and effective type of medical treatment. It presents the opportunity to tackle many physiological conditions and disorders by interacting with the body's own natural neural processes. Neural modulation includes inhibition (e.g. blockage), stimulation, modification, regulation, or therapeutic alteration of activity, electrical or chemical, in the central, peripheral, or autonomic nervous system. By modulating the activity of the nervous system, several different goals may be achieved. For instance, motor neurons may be stimulated at appropriate times to cause muscle contractions. Further, sensory neurons can be blocked to relieve pain or stimulated to provide a signal to a subject or patient. In yet other examples, modulation of the autonomy nervous system may be used to adjust various involuntary physiological parameters, such as heart rate and blood pressure. Neural modulation may provide the opportunity to treat several diseases or physiological conditions. Various devices and techniques have been used in attempts to provide optimum stimulation of a tissue of interest.

One of the conditions to which neural modulation can be applied to is obstructive sleep apnea (OSA), a respiratory disorder characterized by recurrent episodes of partial or complete obstruction of the upper airway during sleep. One of the causes of OSA is the inability of the tongue muscles to resist negative inspiratory pressure in the pharynx due to the sleep-related loss in muscle tone. As the tongue is pulled backwards, it obstructs the upper airway, decreasing ventilation and lowering lung and blood oxygen levels. Stimulation of the hypoglossal nerve for, example, causes the tongue muscles, e.g. the genioglossus muscle, to contract, thereby maintaining an open, unobstructed airway, since the genioglossus muscle is responsible for the forward movement of the tongue as well as for the stiffening of the anterior pharyngeal wall.

### Prior Art

Stimulation devices for use in the detection and treatment of sleep apnea prevention are known in the prior art. For example, US 2003/0153953 A1 discloses a stimulator and a stimulation method for treating sleep apnea. However, the stimulator is dependent on the use of lead wires, which can be subject to fatigue caused by muscle movement.

US 6,240,316 B1 teaches about the treatment of sleep apnea using injectable microstimulators having a tubular housing and no external lead wires. However, said injectable microstimulators do not necessarily stay in the required position, since they are designed for easy implantation through injection rather than for fixed and stable positioning.

US 2014/031840 A1 discloses a device for delivering sleep apnea related implant to location proximate genioglossus muscle, having an implant retainer to hold the implant for implantating genioglossus muscle and maintain the implant in fixation location relative to the tissue beneath skin.

US 2019/126051 A1 discloses a flexible semi-hermetic implantable medical device structure for use in constructing medical devices, having an external flexible polymer structure for encapsulating hermetically sealed components, electrical cable harness and a transition unit.

### Summary of the Disclosed Subject Matter

One of the objectives of the present disclosure is to respond to the disadvantages of the prior art and to provide an improved system for electrical nerve stimulation in a recipient of the stimulation. In particular, an objective of this disclosure is to present a device that is highly durable and ensures accurate nerve stimulation, while reliably staying in a desired position with respect to the tissue to be stimulated.

According to one aspect of the disclosure, an implantable device for use in the treatment of sleep disordered breathing is provided, the device comprising a flexible body and at least one stimulator unit attached to the flexible body, wherein the at least one stimulator unit comprises a receiving antenna, a plurality of passive electric components encapsulated in a hermetically sealed enclosure, and at least one electric conductor in electrical connection with the plurality of passive electric components. Preferably, the electric conductor is a pair of electrodes.

The device as described above allows for an easy and stable positioning of the electrical stimulator in a subject, patient or recipient of the stimulation, e.g. on or around a muscle of the subject. The flexible body may be formed from any suitable, biocompatible material, such that it may be configured to conform to a desired location. The material of the flexible body may include, but is not limited to, silicone, plastic and/or other suitable polymers, copolymers, as well as combinations thereof. The implantable device is implanted at chosen locations within the subject and may be controlled or configured to stimulate muscle and nerve tissue in a constructive manner to help open blocked airways. Providing of a device as described herein eliminates the need for additional anchorage. For example, the flexible implantable device can easily adapt and thus attached to a desired tissue due to its flexibility. Thus, a key aspect of the present disclosure can be regarded as treatment of obstructive sleep apnea by electrically stimulating certain muscles of the orophar-ynx using one or more implantable devices each having one or more stimulator unit in order to contract and thereby pull open the obstructed airway.

The stimulator unit (or "microstimulator") is preferably leadless and receives power signals, stimulation signals and/or recharging signals from a radio frequency (RF) magnetic field generated outside the subject's body. Thus, the stimulator unit comprises the following: a receiving antenna that receives the power signals, stimulation signals and/or recharging signals; a plurality of electric components encapsulated in a hermetically sealed enclosure, wherein the enclosure is preferably made from a made from a biocompatible and/or RF (radio frequency) transparent material; and at least one electric conductor, which is in electrical connection with the plurality of electric components, wherein the at least one electric conductor is configured for application of stimulation signals to a surrounding tissue. With such a stimulator unit, the need for electrical leads to connect the implantable device to another central implanted or external controller can be avoided.

It is possible that each stimulator unit includes at least one processor that may be configured to perform a logic operation. The at least one processor may therefore include one or more integrated circuits, microchips, microcontrollers and microprocessors, which may be all or part of a central processing unit (CPU), a digital signal processor (DSP), a field programmable gate array (FPGA), or any other circuit known to those skilled in the art that may be suitable for executing instructions or performing logic operations. The device as described herein may comprise at least two stimulator units. In such a case, each of the stimulator units can be controlled separately or both stimulator units may be controlled simultaneously, allowing for a singular (unilateral) or a bilateral stimulation of the muscle to stimulated.

As described above, the stimulator unit may be configured to be leadless and thus avoid the necessity of leads or lead wires. An implantable device according to the above has the advantage of comprising only very few parts and therefore being very compact. Moreover, the lack of external lead wires (or other or loose parts) drastically minimalizes the number of parts being subjected to stress and/or fatigue, since lead wires they can be damaged over time as a result of the movement of the stimulated muscle. The stimulator may also be fabricated with a thickness suitable for implantation under a patient's skin. For example, the stimulator may have a thickness of less than 4 mm. Furthermore, the microstimulator may receive electromagnetic signals (such as power signals and stimulation signals) from an external source. When the stimulator unit receives an appropriate signal, it generates a required stimulation pulse releasing energy stored in the capacitor, and then recharging the capacitor between output pulses.

According to one embodiment disclosed herein, the flexible body at least partially consists of silicone. The implantable device may further be characterized in that the flexible body comprises suture holes for connecting the implantable device to a tissue of a subject. Silicone is flexible and biocompatible and facilitates alignment of the implantable device in a desired orientation within a subject's or patient's body. The suture holes provide attachment points for fixing and securing the implantable device at a desired location. Additionally, the flexible body may be formed with a generally triangular, circular, or rectangular shape. The shape of the flexible body may facilitate orientation of the implantable device with respect to a particular nerve or muscle to be modulated. Thus, other regular or irregular shapes may be adopted in order to facilitate implantation in differing parts of the body. The implantable device may additionally be coated with a protective coating. In some embodiments, the protective coating may be made from a flexible material to enable bending along with the flexible body. The encapsulation material of the protective coating may also resist humidity penetration and protect against corrosion.

The implantable device may in particular be configured in such a way that the flexible body has a first arm and a second arm, wherein each arm comprises at least one suture hole. This way, the flexible body of the implantable device may be attached in a desired manner with respect to a particular muscle (e.g. the genioglossus muscle), a nerve within a patient's body, or a surface within a body above a nerve. For example, the first and the second arm may be configured to enable the flexible body to conform at least partially around soft or hard tissue beneath a patient's skin (e.g. nerve, bone, or muscle tissue). With the suture holes placed on the first arm and the second arm of the flexible body, the implantable device may further be secured in a more conforming manner to the desired location, thus keeping the stimulator units of the implantable device in its required position.

The implantable device is configured for implantation proximal to a genioglossus muscle in the vicinity of a hypoglossal nerve of the subject. The hypoglossal nerve, through its lateral branch and medial branch, innervates the muscles of the tongue and other glossal muscles, including the genioglossus muscle and the geniohyoid muscle. The horizontal compartment of the genioglossus muscle is mainly innervated by the medial terminal fibers of the medial branch of the hypoglossal nerve, which diverges from the lateral branch at terminal bifurcation. The distal portion of the medial branch then variegates into the medial terminal fibers. Contraction of the horizontal compartment of the genioglossus muscle may serve to open or maintain a subject's airway. Contraction of other glossal muscles may assist in other functions, such as swallowing, articulation and opening or closing of the airway. Because the hypoglossal nerve innervates several glossal muscles, it may be advantageous for OSA treatment, to confine modulation of the nerve to the medial branch, or maybe even to the medial terminal fibers or the terminal fibers of the nerve. This way, the genioglossus muscle, being most responsible for tongue movement and airway maintenance, may be selectively targeted for contraction inducing neuro-modulation. Alternatively, the horizontal compartment of the genioglossus muscle may be selectively targeted.

It may also be intended that each of the flexible bodies arm has at least one stimulator unit attached to it. This way, more "intelligent", i.e. advanced stimulation strategies may be applied during treatment, since the tissue to be stimulated may be stimulated from at least two different sites. Preferably, the different stimulator units, i.e. the first arm's stimulator and the second arm's stimulator, may be configured to operate independently, thus further increasing the amount of possible stimulation strategies. In a preferred embodiment, the implantable device comprises a flexible body having a first and a second arm, each arm having one microstimulator attached to it, wherein each microstimulator operates independently. Operation of such an "intelligent" stimulation, i.e. the independent stimulator units may be controlled by a logical unit or processor, which may for example be located external to the subject's body.

It is also possible that the receiving antenna is configured to receive power signals and stimulation signals from a transmitting antenna located outside the subject's body via coupling between the transmitting antenna and the receiving antenna. Said coupling of the receiving antenna and an external transmitting may include any interaction between the receiving antenna and the transmitting that causes a signal on the receiving antenna in response to a signal applied to the transmitting antenna. The coupling between the antennas may include capacitive coupling, inductive coupling, radio frequency (RF) coupling and any combinations thereof.

In addition to the above, the stimulator unit comprises at least one electrical circuit for electrically connecting the receiver antenna to the plurality of passive electrical components and back to the at least one electric conductor. The circuit may include conductive materials, such as gold, platinum, titanium, or any biocompatible conductive material or combination of materials. Furthermore, the circuit may include one or more of the following components: resistor, inductor, and/or capacitor.

As part of preferred embodiment and to protect the receiving antenna, the circuit and the passive electrical components from the environment within a patient's body, the at least one stimulator unit comprises a main body. In particular, it may be intended that the receiving antenna is disposed in the main body. The main body may preferably be formed from a biocompatible material comprising, for example, ceramic. It is also possible that the ceramic material of the main body is sintered. In particular, the main body may comprise one or more distinct layers of ceramic, on which a platinum layer is deposited before the ceramic is sintered. That way, the receiving antenna and the electronic circuit may be enclosed or integrated in the main body. Furthermore, the electrodes as well as the passive electric components may be embedded on the main body. Furthermore, the main body may preferably be lenticularly shaped.

Preferably the stimulator unit has a first surface and a second surface, wherein, the stimulator unit comprises a cap disposed on the first surface of the main body, forming the hermetically sealed enclosure. According to further developments, the cap is at least partially made from titanium. The wall of the cap should be very thin, preferably less than 10 microns, as to absorb stress rather than to transfer it to the main body. It is also possible that the cap is attached to the main body through welding of the cap to an annular welding projection disposed on a first surface of the main body. This way, the electric components, which are also attached to the first surface, may be encapsulated in the hermetically sealed enclosure. The welding projection may be made from any biocompatible material, such as indium, gold-tin etc.

In addition to the above, the stimulation unit comprises a plurality of solder pads disposed on the first surface of the stimulation unit and located in the hermetically sealed enclosure, wherein the solder pads are configured for mounting the plurality of passive electric components, and wherein the plurality of passive electric components is soldered, in particular reflow soldered, to the plurality of solder pads.

The implantable device may further be configured such that the at least one electric conductor is disposed on a second surface of the main body. The electrodes may include any suitable shape and orientation on the stimulator so long as the electrodes may be configured to generate an electric field in the body of the patient or subject. The electrodes may also include any suitable conductive material like copper, silver, gold, platinum, iridium, platinum-iridium, platinum-gold, conductive polymers etc., or combinations of conductive materials. In some embodiments the electrodes may include short line electrodes, circular electrodes, and/or circular pairs of electrodes. In one embodiment, the field-generating electrodes may include two distinct electrodes, with one providing an anode and the other providing a cathode. According to a preferred embodiment, the at least one electric conductor is formed as a pair of electrode pads.

According to this disclosure, the receiving antenna can be, but is not limited to, a long-wire antenna, a patch antenna, a helical antenna, a coil antenna, a slow wave antenna, a monopole antenna, a dipole antenna, spiral, oval, rectangular etc. According to a preferred embodiment, however, the receiving antenna has a circular and/or coiled shape. It is furthermore confined to an outer annular area of the stimulation unit, thus increasing its efficiency. According to another embodiment of the implantable device, the hermetically sealed enclosure and the at least one electric conductor are located within an inner circular area of the stimulation unit.

According to the above, a preferred embodiment of the implantable device's stimulator unit or microstimulator comprises: electrode pads embedded on the main body's second surface; a receiver antenna integrated inside the main body at the main body's outer diameter, i.e. within an outer annular area, thus increasing efficiency; a cap welded to an annular welding projection that is disposed on an inner area of the first surface of the main body, providing a hermetically sealed enclosure; solder pads encapsulated in the sealed enclosure, configured to mount the passive electrical components, e.g. via reflow soldering; at least one electronic circuit integrated inside the main body connecting the receiver antenna to the passive electrical components and back to the electrode pads.

### Brief Description of the Drawings

The accompanying drawings, which are incorporated in and constitute a part of this specification, illustrate several examples of the disclosed subject matter. The drawings depicts the following:
- Fig. 1: depicts a schematic view of an implantable device according to an exemplary embodiment;
- Fig. 2: depicts a schematic view of an implantable device according to an alternative embodiment;
- Fig. 3: depicts a schematic cross-sectional view of a stimulator unit according to an exemplary embodiment;
- Fig. 4: depicts a schematic top-view of the stimulator unit according to the embodiment shown in Fig. 3;
- Fig. 5: depicts a schematic bottom-view of the stimulator unit according to the embodiment shown in Fig. 3 or Fig. 4.

### Detailed Description of the Exemplary Embodiments

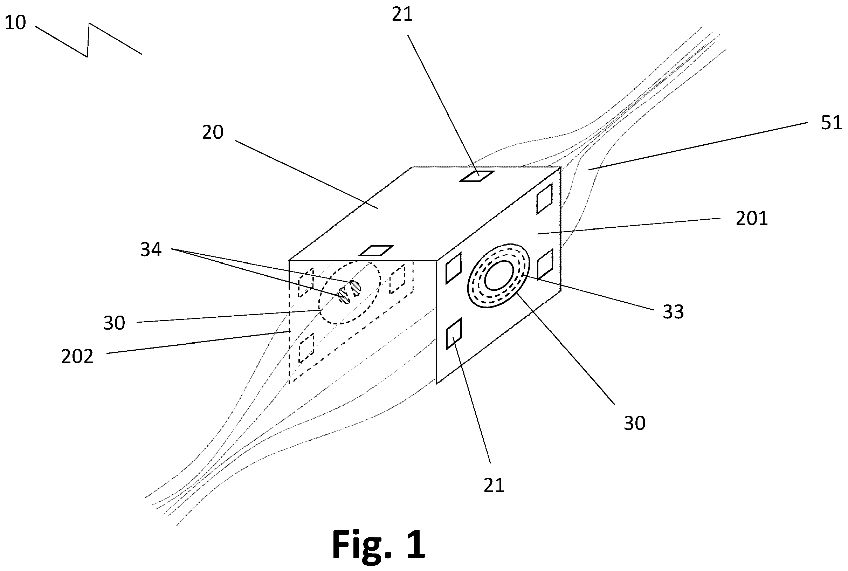

Fig. 1 depicts a schematic view of an implantable device 10 according to an exemplary embodiment. The drawing illustrates an implantable device 10 being attached to a genioglossus muscle 51 of a subject 50. This way, nerves associated with the genioglossal muscle 51 can be modulated, resulting in a stimulation of the subject's 50 tongue. The implantable device 10 as shown in Fig. 1 comprises a flexible body 20 and two stimulators 30 (or microstimulators) unit attached to the flexible body 20, wherein one stimulator unit 30 is attached to a first arm 201 of the flexible body 20 and the other stimulator unit 30 is attached to a second arm 202 of the flexible body 20. The flexible body 20 allows for accurate fitting of the device 10 to the desired tissue. For example, the implantable device 10 according to Fig. 1 is mounted to the genioglossal muscle 51 in a saddle-like shape. This eliminates the need for additional anchorage. Each stimulator unit comprises a receiving antenna 33, a plurality of passive electrical components 31 encapsulated in a hermetically sealed enclosure 32. Furthermore, each stimulator unit 30 comprises at least one electric conductor 34 in electrical connection with the plurality of passive electric components 31. Also, the stimulator units 30 comprise an electrical circuit 35 (not shown) for electrically connecting the receiver antenna 33 to the plurality of passive electrical components 31 and back to the at least one pair of electrodes 34. The stimulator units 30 as depicted in Fig. 1 are of essentially lenticular shape and have a dome-shaped cap 321 attached on a first surface 41 of a main body 40 of the stimulator unit 30. The space encapsulated by the cap 321 is regarded the hermetically sealed enclosure 32 comprising the passive electrical components 31, which are not shown in Fig. 1. With a device 10 as described above, each of stimulator units 30 may be controlled separately, or both simulator units 30 may be controlled simultaneously. This allows for either a singular (unilateral) or a bilateral stimulation of the muscle 51, depending on the chosen treatment.

Having two stimulator units 30, as is the case with the implantable device 10 shown in Fig 1, allows for more advanced stimulation strategies, since the tissue to be stimulated may be stimulated from at least two different sites. Preferably, the two stimulator units 30, i.e. the first arm's 201 stimulator 30 and the second arm's 202 stimulator 30, may be configured to operate independently. This way, the amount of possible stimulation strategies that can be applied to the subject 50 is increased. According to a preferred embodiment shown in Fig. 1, the implantable device 10 is configured in such a way that the two stimulator units 30 are positioned opposite of each other when the flexible body 20 is attached to the muscle tissue 51. Operation of such an "intelligent" stimulation, i.e. of an implantable device 30 having at least two independent stimulator units 30, may be controlled by a processor, which may for example be located external to the subject's 50 body.

Each stimulator unit comprises a receiving antenna 33, which, in case of the embodiment depicted in Fig. 1, is formed as a circular coil antenna 33. The antenna 33 is configured to receive power signals and stimulation signals from a transmitting antenna (not shown) located outside the subject's 50 body via coupling between the transmitting antenna and the receiving antenna 33. Said coupling of the receiving antenna 30 and an external transmitting includes any interaction between the receiving antenna 33 and the transmitting antenna that causes a signal on the receiving antenna 33 in response to a signal applied to the transmitting antenna. The coupling between the antennas may include capacitive coupling, inductive coupling, radio frequency (RF) coupling and any combinations thereof. According to Fig. 1, the receiving antenna 33 is integrated in the main bodies 40 of the stimulator units 30.

According to the above, the stimulator units 30 shown in Fig. 1 are leadless and consist of only few parts. The lack of lead wires drastically minimalizes the number of parts being subjected to stress and/or fatigue, since lead wires they can be damaged over time as a result of the movement of the stimulated muscle. Thus, the stimulator units 30 of Fig. 1 are highly durable and allow for accurate nerve stimulation, while reliably staying in a desired position with respect to the tissue to be stimulated.

The first arm 201 and second arm 202 further facilitate attachment of the implantable device 10 to the desired muscle tissue, since they enable the flexible body 20 to conform at least partially around soft or hard tissue beneath a patient's skin. The flexible body 20 has several suture holes 21 for attaching the implantable unit 10 to the muscle tissue. This way, the flexible body of the implantable device may be attached in a desired manner. With the suture holes 21 placed on the first arm 201 and the second arm 202 of the flexible body 20, the implantable device 10 may be secured more conformingly, thus keeping the stimulator units 30 of the implantable device 10 in their required positions.

The device 10 as shown in Fig. 1 allows for an easy and stable positioning of the stimulator units 30 in a subject 50, patient or recipient of the stimulation, e.g. around a muscle 51 of the subject 50. The flexible body 20 may be formed from any suitable, biocompatible material such that it may be configured to conform to a desired location. The material of the flexible body 20 may preferably consist of silicone.

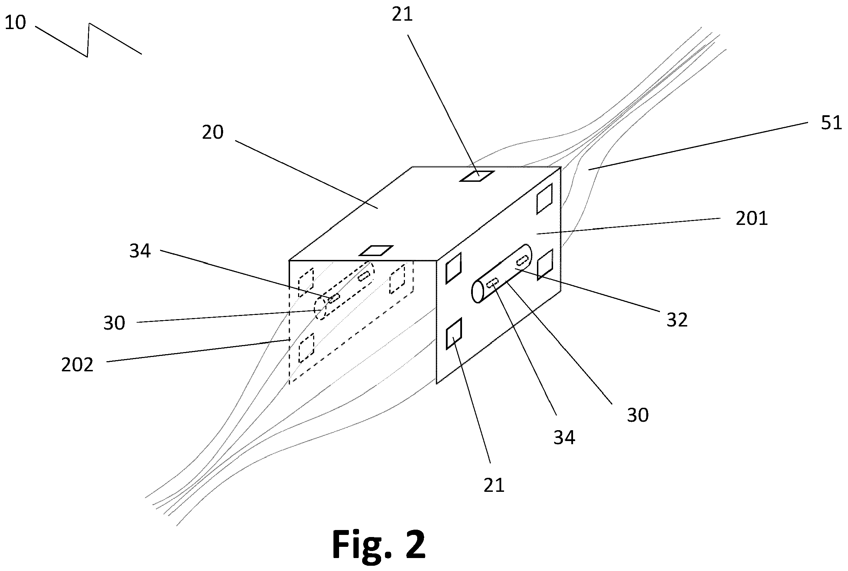

Fig 2. depicts a schematic view of an implantable device 10 according to an alternative embodiment. While being in principle identical to the embodiment shown in Fig. 1, the embodiment depicted in Fig. 2 differs in that all components of the stimulator units 30 are encapsulated in the hermetically sealed enclosure 32, with the exception of the electrode pairs 34, which are disposed on the outside of the enclosure 32 for the application of a stimulation current. Furthermore, the stimulator units 30 are of tubular shape. As is the case with the stimulator units 30 shown in Fig. 1, the stimulator units 30 shown in Fig. 2 are leadless and consist of only few parts. Thus, they are not subjected to fatigue.

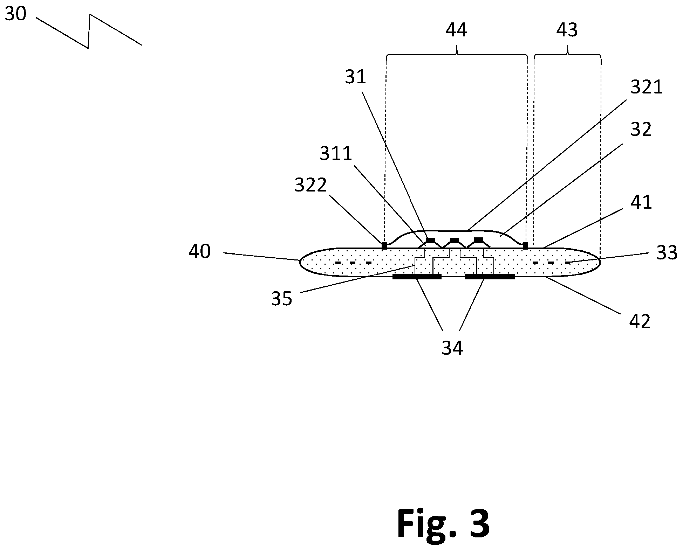

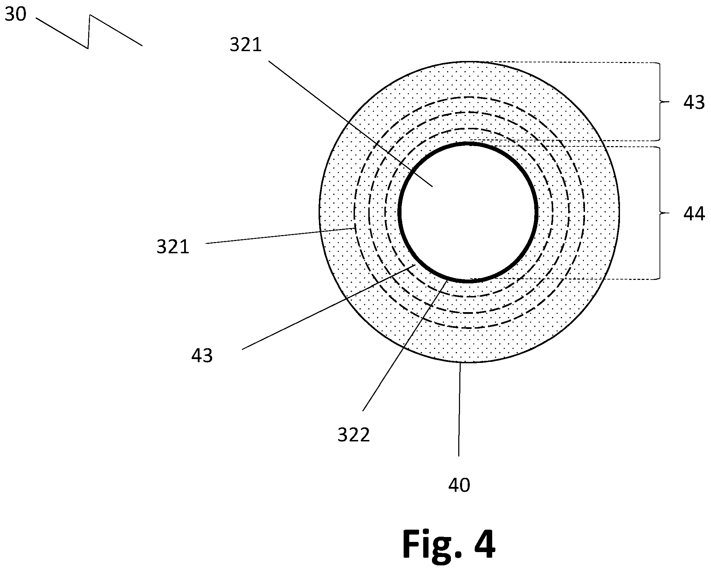

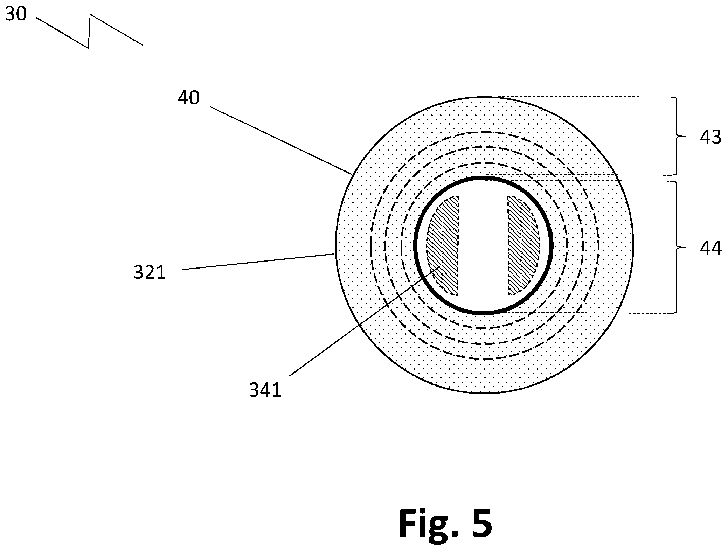

Fig. 3, Fig. 4 and Fig. 5 depict a stimulator unit 30 according to an exemplary unit. More specifically, Fig. 3 shows a schematic cross-sectional view, Fig. 4 shows a schematic top-view and Fig. 5 shows a schematic bottom-view of the stimulator unit 30. The depicted stimulator unit 30 comprises a main body 40 that is substantially made from one or more ceramic layers, on which a platinum layer is deposited before the ceramic is sintered. As shown in Fig. 3, Fig. 4 and Fig. 5, the coiled receiving antenna 33 and the electrical circuit 35 are integrated within the ceramic main body 40 of the stimulator unit 30. That way, the receiving antenna 33 and the electronic circuit 35 may be permanently fixed in the main body 40, avoiding unnecessary movement of intricate parts. The electrical circuit 35 electrically connects the receiver antenna 33 to the plurality of passive electrical components 31 and back to the at least one electric conductor 34. The circuit 35 may include conductive materials, such as gold, platinum, titanium, or any other biocompatible conductive material or combination of materials. Furthermore, the circuit 35 may include one or more of the following components: resistor, inductor, and/or capacitor. The various passive components may be used to connect the passive components least one electric conductor. As shown in Fig. 4 and Fig. 5, the coiled antenna 33 is confined to an outer annular area 43 of the stimulation unit 30, thus increasing its efficiency.

The stimulator unit 30 further comprises a cap 321 attached to a first surface 41 (top side) of the main body 40. The cap 321, which according to the embodiment depicted in Fig. 3 is dome-shaped and made from a material comprising titanium, encapsulates the hermetically sealed enclosure 32. The hermetically sealed enclosure 32 comprises the passive electrical components 31, which are preferably reflow soldered onto a plurality of solder pads 311. The wall of the cap 321 should be very thin, preferably less than 10 microns, as to absorb potential stress rather than to transfer it to the ceramic main body 40. According to the embodiment shown in Fig. 3, Fig. 4 and Fig. 5, the cap 321 is welded to the first surface 41 of the main body 40 using an annular welding projection 322 disposed on the first surface 41 of the main body 40. The welding projection 322 and the solder pads 311 are made from any biocompatible material, such as indium, gold-tin etc. The welding projection 322, the hermetically sealed enclosure 32 as well as the passive components 31 present therein are located within an inner circular area 44 of the stimulation unit 30, which essentially lies inside the outer annular area 43.

As further depicted in Fig. 3, the stimulator unit 30 comprises a electric conductor 34 attached to a second surface 42 (bottom side) of the main body 40. Like the welding projection 322, the hermetically sealed enclosure as well as the passive components 31, the electric conductor 34 is also confined to the inner circular area 44 of the stimulation unit 30. According to a preferred embodiment as well as shown in Fig. 5, the electrodes 34 attached to the second surface of the microstimulator 30 are formed as electrode pads 341.

All features disclosed by the claims, the specification and the figures, as well as all advantages, including constructive particulars, spatial arrangements and methodological steps, can be essential to the invention either on their own or by various combinations with each other.

### List of reference numerals

- 10: Implantable device
- 20: Flexible body
- 21: Suture holes
- 201: First arm
- 202: Second arm

- 30: Stimulator unit
- 31: Plurality of electrical components
- 311: Plurality of solder pads
- 32: Hermetically sealed enclosure
- 321: Cap
- 322: Welding projection
- 33: Receiving antenna
- 34: Electric contductor
- 341: Electrode pad
- 35: Electrical circuit

- 40: Main body
- 41: First surface
- 42: Second surface
- 43: Outer annular area
- 44: Inner circular area

- 50: Subject
- 51: Genioglossus muscle

## Claims

1. An implantable device (10) configured for implantation in a body of subject (50), the device comprising
a flexible body (20), and
at least two stimulator units (30) attached to the flexible body (20),
wherein each stimulator unit (30) comprises
a plurality of electric components (31) encapsulated in a hermetically sealed enclosure (32),
a receiving antenna (33), and
at least one electric conductor (34) in electrical connection with the plurality of electric components (31),
wherein the implantable device is configured for bilateral stimulation.

2. The implantable device (10) according to any of the preceding claims, **characterized in that** the at least one stimulator unit (30) is leadless.

3. The implantable device (10) according to claim 1, **characterized in that** the flexible body (20) at least partly consists of silicone.

4. The implantable device (10) according to one of claims 1 or 2, **characterized in that** the flexible body (20) comprises suture holes (21) for connecting the implantable device (10) to a tissue of a subject (50).

5. The implantable device (10) according to any of the preceding claims, **characterized in that** the implantable device (10) is configured for implantation proximal to a genioglossus muscle in the vicinity of a hypoglossal nerve of the subject (50).

6. The implantable device (10) according to any of the preceding claims, **characterized in that** the flexible body (20) has a first arm (201) and a second arm (202), wherein each arm (201, 202) comprises at least one suture hole (21).

7. The implantable device (10) according claim 6, **characterized in that** each arm (201, 202) of the flexible body (20) has at least one stimulator unit (30) attached to it.

8. The implantable device (10) according to any of the preceding claims, **characterized in that** the receiving antenna (33) is configured to receive power signals and stimulation signals from a transmitting antenna located outside the subject's (50) body via inductive coupling between the transmitting antenna and the receiving antenna (33).

9. The implantable device (10) according to any of the preceding claims, **characterized in that** the stimulator unit (30) comprises at least one electrical circuit (35) for electrically connecting the plurality of passive electrical components (31) to the at least one electric conductor (34).

10. The implantable device (10) according to any of the preceding claims, **characterized in that** the at least one stimulator unit (30) comprises a main body (40).

11. The implantable device (10) according to claim 10, **characterized in that** the receiving antenna (33) is disposed in the main body (40).

12. The implantable device (10) according to one of claims 10 or 11, **characterized in that** the stimulator unit (30) comprises a cap (321) disposed on a first surface (41) of the main body (40), forming the hermetically sealed enclosure (32).

13. The implantable device (10) according to one of claims 11 or 12, **characterized in that** the cap (321) is at least partly made from titanium.

14. The implantable device (10) according to one of claims 12 or 13, **characterized in that** the first surface (41) of the stimulator unit comprises a welding projection (322), wherein the cap (321) is weldable to the welding projection (322).

15. The implantable device (10) according to one of claims 12 to 14, **characterized in that** the stimulation unit (30) comprises a plurality of solder pads (311) disposed on the first surface (41) of the stimulation unit (30) and located in the hermetically sealed enclosure (32), wherein the solder pads (311) are configured for mounting the plurality of electric components (31),

16. The implantable device (10) according to claim 15, **characterized in that** the plurality of electric components (31) is soldered to the plurality of solder pads (311).

17. The implantable device (10) according to one of claims 10 to 16, **characterized in that** the at least one electric conductor (34) is disposed on a second surface (42) of the main body (40).

18. The implantable device (10) according to any of the preceding claims, **characterized in that** the at least one electric conductor (34) is formed as an electrode pad (341).

19. The implantable device (10) according to one of claims 10 to 18, **characterized in that** the main body (40) of the stimulation unit (30) at least partly consists of a ceramic material.

20. The implantable device (10) according to claim 19, **characterized in that** the main body (40) of the stimulation unit (30) further comprises a platinum layer (PL).

21. The implantable device (10) according to one of claims 19 or 20 , **characterized in that** the ceramic material of the main body (40) is sintered.

22. The implantable device (10) according to one of claims 10 to 21, **characterized in that** the receiving antenna (33) has a circular and/or coiled shape and is confined to an outer annular area (43) of the main body (40) of the stimulation unit (30).

23. The implantable device (10) according to one of claims 10 to 22, **characterized in that** the hermetically sealed enclosure (32) and the at least one electric conductor (34) are located within an inner circular area (44) of the main body (40) of the stimulation unit (30).

24. The implantable device (10) according to any of the preceding claims, **characterized in that** the at least one stimulator unit (30) further comprises:
at least one processor configured to perform a logic operation;
at least one electric battery.

## Patentansprüche

1. Implantierbare Vorrichtung (10), die zur Implantation in einen Körper eines Subjekts (50) gestaltet ist, wobei die Vorrichtung Folgendes umfasst
einen flexiblen Körper (20) und
mindestens zwei Stimulatoreinheiten (30), die an dem flexiblen Körper (20) angebracht sind, wobei jede Stimulatoreinheit (30) Folgendes umfasst:
mehrere elektrische Komponenten (31), die in einer hermetisch abgedichteten Umschließung (32) verkapselt sind,
eine Empfangsantenne (33) und
mindestens einen elektrischen Leiter (34) in elektrischer Verbindung mit den mehreren elektrischen Komponenten (31),
wobei die implantierbare Vorrichtung zur beidseitigen Stimulation gestaltet ist.

2. Implantierbare Vorrichtung (10) nach einem der vorhergehenden Ansprüche, **dadurch gekennzeichnet, dass** die mindestens eine Stimulatoreinheit (30) ohne Anschlüsse ist.

3. Implantierbare Vorrichtung (10) nach Anspruch 1, **dadurch gekennzeichnet, dass** der flexible Körper (20) zumindest teilweise aus Silikon besteht.

4. Implantierbare Vorrichtung (10) nach einem der Ansprüche 1 oder 2, **dadurch gekennzeichnet, dass** der flexible Körper (20) Nahtlöcher (21) umfasst, um die implantierbare Vorrichtung (10) mit einem Gewebe des Subjekts (50) zu verbinden.

5. Implantierbare Vorrichtung (10) nach einem der vorhergehenden Ansprüche, **dadurch gekennzeichnet, dass** die implantierbare Vorrichtung (10) für die Implantation proximal zu einem Kinn-Zungen-Muskel in der Nähe eines Unterzungennervs des Subjekts (50) gestaltet ist.

6. Implantierbare Vorrichtung (10) nach einem der vorhergehenden Ansprüche, **dadurch gekennzeichnet, dass** der flexible Körper (20) einen ersten Arm (201) und einen zweiten Arm (202) aufweist, wobei jeder Arm (201, 202) mindestens ein Nahtloch (21) aufweist.

7. Implantierbare Vorrichtung (10) nach Anspruch 6, **dadurch gekennzeichnet, dass** jeder Arm (201, 202) des flexiblen Körpers (20) mindestens eine an ihm angebrachte Stimulatoreinheit (30) aufweist.

8. Implantierbare Vorrichtung (10) nach einem der vorhergehenden Ansprüche, **dadurch gekennzeichnet, dass** die Empfangsantenne (33) dafür gestaltet ist, Energiesignale und Stimulationssignale von einer Sendeantenne, die sich außerhalb des Körpers des Subjekts (50) befindet, über eine induktive Kopplung zwischen der Sendeantenne und der Empfangsantenne (33) zu empfangen.

9. Implantierbare Vorrichtung (10) nach einem der vorhergehenden Ansprüche, **dadurch gekennzeichnet, dass** die Stimulatoreinheit (30) mindestens eine elektrische Schaltung (35) zur elektrischen Verbindung der mehreren passiven elektrischen Komponenten (31) mit dem mindestens einen elektrischen Leiter (34) umfasst.

10. Implantierbare Vorrichtung (10) nach einem der vorhergehenden Ansprüche, **dadurch gekennzeichnet, dass** die mindestens eine Stimulatoreinheit (30) einen Hauptkörper (40) umfasst.

11. Implantierbare Vorrichtung (10) nach Anspruch 10, **dadurch gekennzeichnet, dass** die Empfangsantenne (33) in dem Hauptkörper (40) angeordnet ist.

12. Implantierbare Vorrichtung (10) nach einem der Ansprüche 10 oder 11, **dadurch gekennzeichnet, dass** die Stimulatoreinheit (30) eine Kappe (321) umfasst, die auf einer ersten Oberfläche des Hauptkörpers (40) angeordnet ist, wodurch die hermetisch abgedichtete Umschließung (32) gebildet ist.

13. Implantierbare Vorrichtung (10) nach einem der Ansprüche 11 oder 12, **dadurch gekennzeichnet, dass** die Kappe (321) zumindest teilweise aus Titan besteht.

14. Implantierbare Vorrichtung (10) nach einem der Ansprüche 12 oder 13, **dadurch gekennzeichnet, dass** die erste Oberfläche (41) der Stimulatoreinheit einen Schweißvorsprung (322) umfasst, wobei die Kappe (321) an den Schweißvorsprung (322) schweißbar ist.

15. Implantierbare Vorrichtung (10) nach einem der Ansprüche 12 bis 14, **dadurch gekennzeichnet, dass** die Stimulatoreinheit (30) mehrere Lötpads (311) umfasst, die auf der ersten Oberfläche (41) der Stimulatoreinheit (30) angeordnet sind und sich in der hermetisch abgedichteten Umschließung (32) befinden, wobei die Lötpads (311) zum Montieren der mehreren elektrischen Komponenten (31) gestaltet sind.

16. Implantierbare Vorrichtung (10) nach Anspruch 15, **dadurch gekennzeichnet, dass** die mehreren elektrischen Komponenten (31) an die mehreren Lötpads (311) gelötet sind.

17. Implantierbare Vorrichtung (10) nach einem der Ansprüche 10 bis 16, **dadurch gekennzeichnet, dass** der mindestens eine elektrische Leiter (34) auf einer zweiten Oberfläche (42) des Hauptkörpers (40) angeordnet ist.

18. Implantierbare Vorrichtung (10) nach einem der vorhergehenden Ansprüche, **dadurch gekennzeichnet, dass** der mindestens eine elektrische Leiter (34) als ein Elektrodenpad (341) gebildet ist.

19. Implantierbare Vorrichtung (10) nach einem der Ansprüche 10 bis 18, **dadurch gekennzeichnet, dass** der Hauptkörper (40) der Stimulatoreinheit (30) zumindest teilweise aus einem keramischen Material besteht.

20. Implantierbare Vorrichtung (10) nach Anspruch 19, **dadurch gekennzeichnet, dass** der Hauptkörper (40) der Stimulatoreinheit (30) ferner eine Platinschicht (PL) umfasst.

21. Implantierbare Vorrichtung (10) nach einem der Ansprüche 19 oder 20, **dadurch gekennzeichnet, dass** das keramische Material des Hauptkörpers (40) gesintert ist.

22. Implantierbare Vorrichtung (10) nach einem der Ansprüche 10 bis 21, **dadurch gekennzeichnet, dass** die Empfangsantenne (33) eine Kreis- und/oder Spulenform aufweist und auf einen äußeren kranzförmigen Bereich (43) des Hauptkörpers (40) der Stimulatoreinheit (30) begrenzt ist.

23. Implantierbare Vorrichtung (10) nach einem der Ansprüche 10 bis 22, **dadurch gekennzeichnet, dass** sich die hermetisch abgedichtete Umschließung (32) und der mindestens eine elektrische Leiter (34) in einem inneren kreisförmigen Bereich (44) des Hauptkörpers (40) der Stimulatoreinheit (30) befinden.

24. Implantierbare Vorrichtung (10) nach einem der vorhergehenden Ansprüche, **dadurch gekennzeichnet, dass** die mindestens eine Stimulatoreinheit (30) ferner Folgendes umfasst:
mindesten einen Prozessor, der dafür gestaltet ist, eine logische Operation durchzuführen; mindestens eine elektrische Batterie.

## Revendications

1. Dispositif implantable (10) configuré pour être implanté dans le corps d'un sujet (50), le dispositif comprenant
un corps flexible (20), et
au moins deux unités de stimulation (30) fixées au corps flexible (20), dans lequel chaque unité de stimulation (30) comprend
une pluralité de composants électriques (31) encapsulés dans une enceinte hermétiquement close (32),
une antenne de réception (33), et
au moins un conducteur électrique (34) en liaison électrique avec la pluralité de composants électriques (31),
dans lequel le dispositif implantable est configuré pour une stimulation bilatérale.

2. Dispositif implantable (10) selon l'une quelconque des revendications précédentes, **caractérisé en ce que** l'au moins une unité de stimulation (30) est sans fil.

3. Dispositif implantable (10) selon la revendication 1, **caractérisé en ce que** le corps flexible (20) est constitué au moins en partie de silicone.

4. Dispositif implantable (10) selon l'une des revendications 1 ou 2, **caractérisé en ce que** le corps flexible (20) comprend des trous de suture (21) pour relier le dispositif implantable (10) à un tissu d'un sujet (50).

5. Dispositif implantable (10) selon l'une quelconque des revendications précédentes, **caractérisé en ce que** le dispositif implantable (10) est configuré pour être implanté à proximité d'un muscle génioglosse au voisinage d'un nerf hypoglosse du sujet (50).

6. Dispositif implantable (10) selon l'une quelconque des revendications précédentes, **caractérisé en ce que** le corps flexible (20) possède un premier bras (201) et un second bras (202), dans lequel chaque bras (201, 202) comprend au moins un trou de suture (21).

7. Dispositif implantable (10) selon la revendication 6, **caractérisé en ce que** chaque bras (201, 202) du corps flexible (20) possède au moins une unité de stimulation (30) qui est fixée à celui-ci.

8. Dispositif implantable (10) selon l'une quelconque des revendications précédentes, **caractérisé en ce que** l'antenne de réception (33) est configurée pour recevoir des signaux de puissance et des signaux de stimulation à partir d'une antenne d'émission située à l'extérieur du corps du sujet (50) par l'intermédiaire d'un couplage inductif entre l'antenne d'émission et l'antenne de réception (33).

9. Dispositif implantable (10) selon l'une quelconque des revendications précédentes, **caractérisé en ce que** l'unité de stimulation (30) comprend au moins un circuit électrique (35) pour relier électriquement la pluralité de composants électriques passifs (31) à l'au moins un conducteur électrique (34).

10. Dispositif implantable (10) selon l'une quelconque des revendications précédentes, **caractérisé en ce que** l'au moins une unité de stimulation (30) comprend un corps principal (40).

11. Dispositif implantable (10) selon la revendication 10, **caractérisé en ce que** l'antenne de réception (33) est disposée dans le corps principal (40).

12. Dispositif implantable (10) selon l'une des revendications 10 ou 11, **caractérisé en ce que** l'unité de stimulation (30) comprend un capuchon (321) disposé sur une première surface (41) du corps principal (40), formant l'enceinte hermétiquement close (32).

13. Dispositif implantable (10) selon l'une des revendications 11 ou 12, **caractérisé en ce que** le capuchon (321) est au moins en partie constitué de titane.

14. Dispositif implantable (10) selon l'une des revendications 12 ou 13, **caractérisé en ce que** la première surface (41) de l'unité de stimulation comprend une protubérance de soudage (322), le capuchon (321) pouvant être soudé à la protubérance de soudage (322).

15. Dispositif implantable (10) selon l'une des revendications 12 à 14, **caractérisé en ce que** l'unité de stimulation (30) comprend une pluralité de plages de soudure (311) disposées sur la première surface (41) de l'unité de stimulation (30) et situées dans l'enceinte hermétiquement close (32), dans lequel les plages de soudure (311) sont configurées pour monter la pluralité de composants électriques (31).

16. Dispositif implantable (10) selon la revendication 15, **caractérisé en ce que** la pluralité de composants électriques (31) est soudée à la pluralité de plages de soudure (311).

17. Dispositif implantable (10) selon l'une des revendications 10 à 16, **caractérisé en ce que** l'au moins un conducteur électrique (34) est disposé sur une seconde surface (42) du corps principal (40).

18. Dispositif implantable (10) selon l'une quelconque des revendications précédentes, **caractérisé en ce que** l'au moins un conducteur électrique (34) se présente sous la forme d'une pastille d'électrode (341).

19. Dispositif implantable (10) selon l'une des revendications 10 à 18, **caractérisé en ce que** le corps principal (40) de l'unité de stimulation (30) est constitué au moins en partie d'un matériau céramique.

20. Dispositif implantable (10) selon la revendication 19, **caractérisé en ce que** le corps principal (40) de l'unité de stimulation (30) comprend en outre une couche de platine (PL).

21. Dispositif implantable (10) selon l'une des revendications 19 ou 20, **caractérisé en ce que** le matériau céramique du corps principal (40) est fritté.

22. Dispositif implantable (10) selon l'une des revendications 10 à 21, **caractérisé en ce que** l'antenne de réception (33) a une forme circulaire et/ou spiralée et est confinée dans une zone annulaire externe (43) du corps principal (40) de l'unité de stimulation (30).

23. Dispositif implantable (10) selon l'une des revendications 10 à 22, **caractérisé en ce que** l'enceinte hermétiquement close (32) et l'au moins un conducteur électrique (34) sont situés à l'intérieur d'une zone circulaire intérieure (44) du corps principal (40) de l'unité de stimulation (30).

24. Dispositif implantable (10) selon l'une quelconque des revendications précédentes, **caractérisé en ce que** l'au moins une unité de stimulation (30) comprend en outre :
au moins un processeur configuré pour effectuer une opération logique; au moins une batterie électrique
